# EUROPEAN PATENT SPECIFICATION

(11) **EP 3 454 853 B1**
(45) Date of publication and mention of the grant of the patent: **18.05.2022**
(21) Application number: 17796695.9
(22) Date of filing: 09.05.2017
(51) Int. Cl.: A61K 31/44, A61K 31/135, A61K 31/4439, A61K 45/06, A61K 31/437, A61K 31/485, A61P 25/24, A61P 25/32, A61K 31/185, A61K 31/197, A61K 31/265, A61K 31/357, A61K 31/4015, A61K 31/4178, A61K 31/4985, A61K 31/517, A61K 31/554

(54) **TREATMENT OF ALCOHOLISM AND DEPRESSION USING IBUDILAST**
BEHANDLUNG VON ALKOHOLISMUS UND DEPRESSION UNTER VERWENDUNG VON IBUDILAST
TRAITEMENT DE L'ALCOOLISME ET DE LA DÉPRESSION AVEC DE L'IBUDILAST

(30) Priority: 10.05.2016 US 201662333990 P
(43) Date of publication of application: 20.03.2019
(73) Proprietor: MediciNova, Inc., La Jolla, CA 92037 (US)
(72) Inventor: IWAKI, Yuichi, La Jolla California 92037 (US)
(74) Representative: Nederlandsch Octrooibureau
(86) International application number: PCT/US2017/031775
(87) International publication number: WO 2017/196857

(56) References cited:
- US-A1- 2007 072 899
- US-A1- 2009 028 816
- US-A1- 2009 062 330
- US-A1- 2015 051 191
- US-A1- 2015 051 191
- KURIA et al.: "The Association between Alcohol Dependence and Depression before and after Treatment for Alcohol Dependence", ISRN Psychiatry, vol. 2012, 2012, pages 1-6, XP055441118,
- "Mayo Clinic Staff' Alcohol use disorder", Mayo Clinic, 25 July 2015 (2015-07-25), pages 1-3, XP055582278, Retrieved from the Internet: URL:http://www.mayoclinic.org/diseases-con ditions/alcohol-use- disorder/basics/definition/con-20020866 [retrieved on 2017-07-10]
- THOMAS M KELLY ET AL: "Treatment of substance abusing patients with comorbid psychiatric disorders", ADDICTIVE BEHAVIORS, vol. 37, no. 1, 1 January 2012 (2012-01-01), pages 11-24, XP028318888, ISSN: 0306-4603, DOI: 10.1016/J.ADDBEH.2011.09.010 [retrieved on 2011-09-14]

## Description

### BACKGROUND OF THE INVENTION

This application claims priority under 35 U.S.C. § 119(e) to U.S. Provisional Application No. 62/333,990, filed May 10, 2016.

Excessive consumption of alcohol is a major health concern globally. Alcoholism is considered to be a chronic disease, a drug addiction, a learned response to crisis, a symptom of an underlying psychological or physical disorder, or a combination of these factors and is marked by repeated alcohol use despite a host of negative, physical and psychosocial effects.

Most approaches to the treatment of alcohol use disorder (AUD) require the alcoholic person to recognize his/her illness and to abstain from alcohol. Treatment programs can include combinations of: psychological rehabilitative treatments, organized self-help groups, aversion therapy based on behavior modification, injections of vitamins or hormones, and the use of abstinence-maintaining drugs.

Many patients diagnosed with AUD also exhibit symptoms of depression and/or dysphoric mood.

To date, only a few agents are approved by the Food and Drug Administration (FDA) for the treatment of alcoholism and these agents are only modestly effective. While drugs such as acamprosate (CAMPRAL), ondansetron (ZOFRAN), naltrexone (VIVITROL), disulfiram (ANTABUSE) and calcium citrate cyanamide are used as therapeutics for treating alcoholism, these therapeutic agents have drawbacks related to their efficacy and associated side effects. Provided herein are methods and compositions related to the use of ibudilast to treat patients diagnosed with alcohol use disorder and exhibiting symptoms of depression and/or dysphoric mood.

US 2015/051191 discloses the oral use of ibudilast for treating alcoholism. According to US 2015/051191, withdrawal from alcohol typically results in a condition marked by feelings of nervousness, hyperexcitability, sleep disturbances and dysphoric mood and treatment of alcoholism with ibudilast lowers or eliminates *withdrawal associated* behavioural changes. US 2015/051191 does not relate to depression.

US 2009/028816 teaches the use of ibudilast for treating affective disorders, such as depression, psychosis, or anxiety. US 2009/028816 provides no disclosure regarding AUD and it does not contain any data showing the effect of ibudilast in the treatment of any such disorder.

### SUMMARY OF THE INVENTION

In one aspect, provided herein is ibudilast or a pharmaceutically acceptable salt thereof, for use in a method of treating a patient diagnosed with alcohol use disorder (AUD), said method comprising administering to a patient diagnosed with AUD a therapeutically effective amount of ibudilast or a pharmaceutically acceptable salt thereof, in which the patient also exhibits symptoms of depression.

In some embodiments, the patient exhibits symptoms of mild, moderate, and/or severe depression.

In some embodiments, ibudilast can be administered orally as a therapeutically effective daily dose from about 3 mg to about 200 mg, for instance a therapeutically effective daily dose of 100 mg or 200 mg. As used herein, the amounts refers to amounts of ibudilast or the free base equivalent amount thereof of a pharmaceutically acceptable salt of ibudilast. The therapeutically effective daily dose may be administered as a single dose or it can be divided in two doses of 50 mg each or two doses of 100 mg each. Alternatively, the therapeutically effective daily dose may be divided into three or four doses.

For certain embodiments, the therapeutically effective daily dose is 100 mg or less and is divided into two doses. One or more other therapeutic agents may be administered along with ibudilast. Non-limiting examples of other therapeutic agents suitable for the treatment of AUD and depression and/or dysphoric mood include a psychiatric agent and/ or a therapeutic agent known to have utility in AUD

### BRIEF DESCRIPTION OF THE DRAWINGS

**FIG. 1** graphically illustrates the mean ethanol intakes for P (upper panels) and HAD1 (lower panels) rats during the maintenance (left side of panel) and relapse (right side of panel) test-phases. ^{∗} indicates the respective dose differed significantly from vehicle (p< 0.05). Grey bar indicates the vehicle value.
**FIG. 2** graphically illustrates the mean ethanol intakes for Chronic Intermittent Ethanol (EtOH) exposed mice and control (CTL) mice during Test Cycles 7, 8, 9. For comparison purposes, the horizontal dashed line indicates mean ethanol intake by vehicle-treated CTL mice during test cycle 7. ^{∗} indicates less ethanol intake compared to vehicle-treated mice (p< 0.05). # indicates greater intake than CTL group (p< 0.05).
**FIG. 3** is a flow chart illustrating human Phase I clinical study.
**FIG. 4** graphically illustrates that ibudilast, but not placebo, significantly decreased basal, daily AUQ craving over the course of the Phase I clinical study.
**FIG. 5** graphically illustrates that ibudilast did not affect cue- and stress-induced AUQ craving during the human Phase I clinical study.
**FIG. 6** graphically illustrates that ibudilast increased positive mood during both the cue reactivity and stress procedures during the human Phase I clinical study.
**FIG. 7** graphically illustrates that ibudilast did not affect cortisol reactivity to a stressor but did produce a modest increase in overall cortisol level vs. placebo during the human Phase I clinical study.

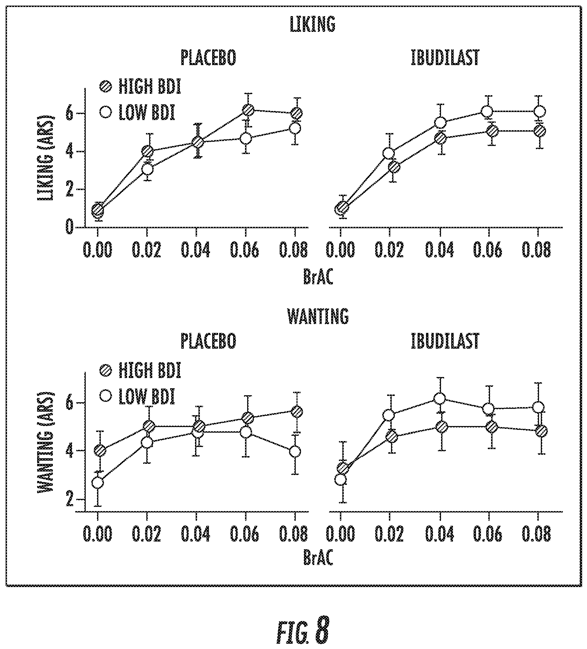

**FIG. 8** graphically illustrates that ibudilast and levels of depressive symptomatology affect alcohol "liking" and "wanting."

### DETAILED DESCRIPTION

The practice of the present technology will employ, unless otherwise indicated, conventional methods of chemistry, biochemistry, and pharmacology, within the skill of the art. Such techniques are explained fully in the literature. See, e.g.; A.L. Lehninger, Biochemistry (Worth Publishers, Inc., current addition); Morrison and Boyd, Organic Chemistry (Allyn and Bacon, Inc., current addition); J. March, Advanced Organic Chemistry (McGraw Hill, current addition); Remington: The Science and Practice of Pharmacy, A. Gennaro, Ed., 20th Ed.; Goodman & Gilman The Pharmacological Basis of Therapeutics, J. Griffith Hardman, L. L. Limbird, A. Gilman, 10th Ed.

As used herein, and in the appended claims, the singular forms "a," "an" and "the" include plural references unless the context clearly dictates otherwise.

"Administering" or "administration of' a drug to a patient (and grammatical equivalents of this phrase) includes both direct administration, including self-administration, and indirect administration, including the act of prescribing a drug. For example, as used herein, a physician who instructs a patient to self-administer a drug and/or provides a patient with a prescription for a drug is administering the drug to the patient.

"Comprising" shall mean that the methods and compositions include the recited elements, but not exclude others. "Consisting essentially of' when used to define methods and compositions, shall mean excluding other elements of any essential significance to the combination for the stated purpose. "Consisting of' shall mean excluding more than trace elements of other ingredients and substantial method steps for administering the compositions utilized or provided herein or process steps to produce a composition or achieve an intended result. Embodiments defined by each of these transitional terms and phrases are within the scope of this technology.

"Ibudilast" refers to a compound of formula:

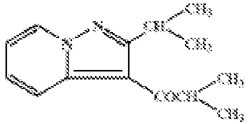

"Pharmaceutically acceptable" refers to safe and non-toxic for the methods and compositions provided herein, e.g., for *in vitro* or *in vivo* administration, preferably to mammals.

A "pharmaceutically acceptable salt" is a pharmaceutically acceptable, organic or inorganic acid or base salt of a compound. Representative pharmaceutically acceptable salts include, *e.g.*, alkali metal salts, alkali earth salts, ammonium salts, watersoluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts. A pharmaceutically acceptable salt can have more than one charged atom in its structure. In this instance the pharmaceutically acceptable salt can have multiple counterions. Thus, a pharmaceutically acceptable salt can have one or more charged atoms and/or one or more counterions.

The terms "treat", "treating" and "treatment" refer to the lowering or reduction of, or amelioration or eradication of a disease or one or more symptoms associated with a disease. In certain embodiments, such terms refer to minimizing the spread or worsening of the disease resulting from the administration of one or more prophylactic or therapeutic agents to a patient with such a disease. For purposes of the various aspects and embodiments provided herein, "treatment" includes, but are not limited to, reduction, alleviation, or amelioration of one or more manifestations of or negative effects of the disease or condition treated, improvement in one or more clinical outcomes, diminishment of extent of disease, delay or slowing of disease progression, amelioration, palliation, or stabilization of the disease state, and other beneficial results described herein.

The term "effective amount" refers to an amount of a compound sufficient to provide a therapeutic or prophylactic benefit in the treatment or prevention of a disease or to delay or minimize symptoms associated with a disease. Further, a therapeutically effective amount with respect to a compound utilized herein means that amount of the compound alone, or in combination with other therapies, that provides a therapeutic benefit in the treatment or prevention of a disease. Used in connection with a compound utilized herein, the term can encompass an amount that improves overall therapy, reduces or avoids symptoms or causes of disease, or enhances the therapeutic efficacy of or synergies with another therapeutic agent. The full therapeutic effect does not necessarily occur by administration of one dose (or dosage), and may occur only after administration of a series of doses. Thus, an effective amount may be administered in one or more administrations.

A "subject" or "patient" may include an animal, such as a human, cow, horse, sheep, lamb, pig, chicken, turkey, quail, cat, dog, mouse, rat, rabbit or guinea pig. The animal can be a mammal such as a non-primate and a primate (*e.g.*, monkey or human). In one embodiment, the patient is a human.

"Alcoholism" or "alcohol dependence" or "alcohol use disorder" is a chronic and often progressive disease that includes problems controlling one's drinking, being preoccupied with alcohol, continuing to use alcohol even when it causes problems, having to drink more to get the same effect (physical dependence), or having withdrawal symptoms when you rapidly decrease or stop drinking. Alcoholism is considered to be a chronic disease, a drug addiction, a learned response to crisis, a symptom of an underlying psychological or physical disorder, or a combination of these factors and is marked by repeated alcohol use despite a host of negative physical and psychosocial effects. Signs and symptoms of alcoholism include, without limitation: to be unable to limit the amount of alcohol one drinks; feel a strong need or compulsion to drink; develop tolerance to alcohol so that you need more to feel its effects; drink alone or hide one' drinking; experience physical withdrawal symptoms - such as nausea, sweating and shaking - when one does not drink; forget conversations or commitments, sometimes referred to as a "black out;" make a ritual of having drinks at certain times and become annoyed when this ritual is disturbed or questioned; be irritable when your usual drinking time nears, especially if alcohol is not available; keep alcohol in unlikely places at home, at work or in one's car; gulp drinks, order doubles or become drunk intentionally to feel good, or drink to feel "normal;" have legal problems or problems with relationships, employment or finances due to drinking; and lose interest in activities and hobbies that used to bring you pleasure.

The results described below demonstrate ibudilast's ability to decrease voluntary ethanol consumption, under blind testing conditions, in selectively bred alcohol-preferring (P) and high-alcohol-drinking (HAD1) rats. The ability of ibudilast to treat alcohol dependence using a mouse model of ethanol dependence was tested; the which model involved repeated cycles of chronic intermittent ethanol (CIE) exposure (see Litten et al. Addict. Biol., 17:513-527, (2012). Because elevated intake of alcohol is characteristic of animals used in each of the above mentioned models and such an increase in alcohol intake is contemplated to result from biological mechanisms relevant to human alcohol dependence, the above-mentioned models are contemplated to be suitable for testing the utility of ibudilast for reducing alcohol intake in humans. See Egli et al., Addict. Biol., 10:309-319, (2005)

Results using alcohol-preferring and high-alcohol-drinking rats indicate that ibudilast reduces ethanol intake in rats in both groups by approximately 50% as compared to control rats. As further explained below in the Examples section, the test was designed to measure the ability of ibudilast to decrease voluntary ethanol consumption in four phases: an initial maintenance test phase, an alcohol drinking recovery phase, an alcohol deprivation phase and an alcohol reintroduction (relapse) phase.

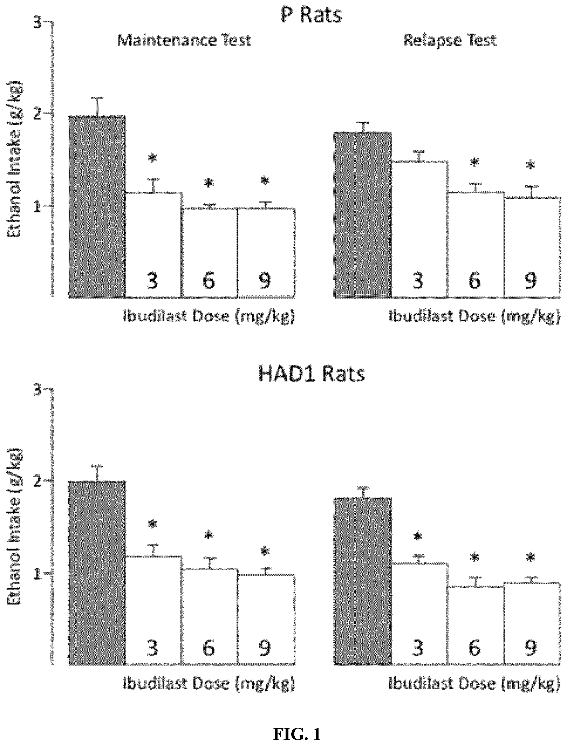

As illustrated in **FIG. 1**, ibudilast decreased ethanol intake in a dose dependent manner in both alcohol-preferring and high-alcohol-drinking rats during the maintenance and relapse phases. Ethanol intake levels were elevated, however, during the recovery phase when no treatment is administered.

A correlation between the dose of ibudilast and number of days over which treatment was administered is also observed for alcohol-preferring and high-alcohol-drinking rats. The ability of ibudilast to reduced intake of ethanol was strongest on the first day of the relapse phase and ethanol intake levels increased gradually in both groups of rats on days 2-5 of the relapse test phase.

Ibudilast also reduced ethanol intake in adult male C57BL/6J mice model of alcohol dependence. Briefly, mice were divided into test (ethanol dependent) and control (non-ethanol dependent) groups. Mice in the former group were exposed to chronic intermittent ethanol (CIE) vapor (16 hr/day x 4 days), and then forced to abstain from alcohol for 72 hours. Mice were then permitted access to ethanol for 2h/day for a 5-day test period. This pattern of weekly CIE exposures followed by 5-day test periods was repeated for 9 cycles. Mice in the control group were treated in the same manner, except these animals were exposed to air rather than ethanol vapors. Mice from both groups were further divided into four sub-groups with animals receiving 0 mg/Kg, 3 mg/Kg, 6 mg/Kg or 12 mg/Kg dose of ibudilast.

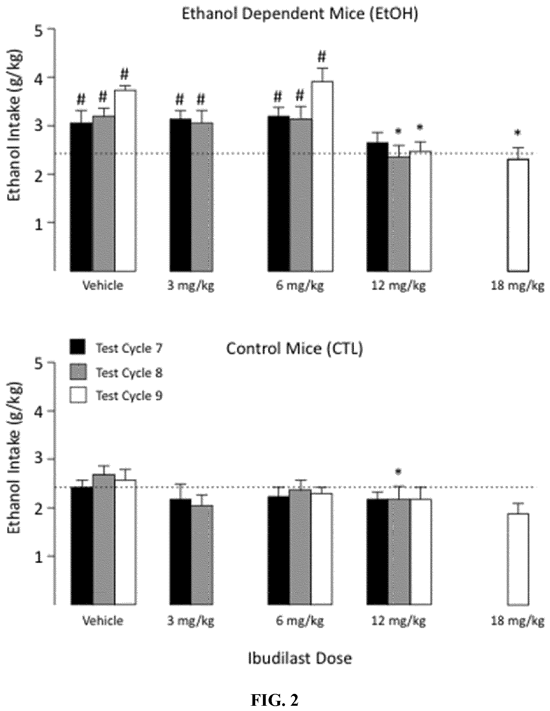

As illustrated in **FIG. 2**, ibudilast reduced ethanol intake in both control and test groups. The therapeutic effect of ibudilast was stronger in the test group and the therapeutic efficacy increased over repeated testing and drug treatment cycles starting from cycle 7. Surprisingly, at the higher doses, ibudilast decreased ethanol intake of mice in the test group to ethanol intake levels below those for mice in the control group.

In one aspect, provided herein is a method of treating a patient diagnosed with alcohol use disorder (AUD) comprising administering to the patient diagnosed with AUD a therapeutically effective amount of ibudilast or a pharmaceutically acceptable salt thereof, in which the patient also exhibits symptoms of depression

In some embodiments, the patient diagnosed with AUD exhibits symptoms of mild depression. In some embodiments, the patient diagnosed with AUD exhibits symptoms of moderate depression. In some embodiments, the patient diagnosed with AUD exhibits symptoms of severe depression.

In some embodiments, the patient diagnosed with AUD also exhibits symptoms of higher dysphoric mood. In some embodiments, the patient diagnosed with AUD also exhibits symptoms of moderate dysphoric mood. In some embodiments, the patient diagnosed with AUD also exhibits symptoms of lower dysphoric mood.

In addition to ibudilast, the treatment of AUD and depression may optionally include one or more other therapeutic agents. These agents can be administered together with ibudilast or separately.

In some embodiments, the other therapeutic agent can be a psychiatric therapeutic agent. Examples of psychiatric therapeutic agents include but are not limited to lithium, sertraline, citalopram, carbamazepine, amisulpride, clomipramine, gabapentin, amitriptyline, doxepin, lamotrigine, amoxapine, escitalopram, levetiracetam, agomelatin, fluoxetine, bupropion, fluvoxamine, oxcarbazepine, imipramine, topiramate, clomipramine, mirtazapine, sodium valproate, desipramine, paroxetine, divalproex sodium, desvenlafaxine, sodium valproate, duloxetine, escitalopram, moclobemide, nortripytyline, phenelzine, reboxetine, tianeptine, tranylcypromine, trazodone, venlafaxine, vilazodone, vortioxetine, or any combination of one or more of the foregoing.

In some embodiments, the other therapeutic agent is approved and known to have utility in AUD In some embodiments, the other therapeutic agent can be acamprosate, baclofen, naltrexone, quetiapine, disulfiram, ondansetron, varenicline, topiramate, prazocin, sertraline, levatiracetam, or any combination of one or more of the foregoing. In some embodiments, the other therapeutic agent is naltrexone.

In another aspect, a pharmaceutical composition is provided, the composition including a therapeutically effective amount of ibudilast or a pharmaceutically acceptable prodrug, or salt thereof and a pharmaceutically acceptable carrier for treating AUD and depression.

Pharmaceutical compositions provided herein encompass, in some embodiments, formulations suitable for systemic administration. Oral dosage forms include tablets, lozenges, capsules, syrups, oral suspensions, emulsions, granules, and pellets. Alternative formulations include transdermal patches, powders or lyophilates that can be reconstituted with the help of a suitable diluent. Examples of suitable diluents for reconstituting solid compositions, e.g., prior to injection, include bacteriostatic water for injection, dextrose 5% in water, phosphate-buffered saline, Ringer's solution, saline, sterile water, deionized water, and combinations thereof.

Formulations suitable for parenteral administration include aqueous and non-aqueous isotonic sterile solutions suitable for injection, as well as aqueous and non-aqueous sterile suspensions. Parenteral formulations provided herein are optionally contained in unit-dose or multi-dose sealed containers, for example, ampoules and vials, and may be stored in a freeze-dried (lyophilized) condition requiring only the addition of the sterile liquid carrier, for example, water for injections, immediately prior to use.

A formulation provided herein is a sustained release formulation suitable for oral or parenteral delivery. The sustained release compositions can contain ibudilast alone or a combination of ibudilast and a second drug. For such compositions, each drug is released or absorbed slowly over time, when compared to a non-sustained release formulation. Sustained release formulations may employ pro-drug forms of the active agent, delayed-release drug delivery systems such as coatings, liposomes, polymer matrices, or hydrogels.

The pharmaceutically acceptable carrier material can be an organic or inorganic inert carrier material, for example one that is suitable for oral administration. Suitable carriers include water, gelatin, gum arabic, lactose, starch, magnesium stearate, talc, vegetable oils, polyalkylene-glycols, petroleum jelly and the like that are used to manufacture tablets, capsules, lozenges and the like. Furthermore, the pharmaceutical preparations may also contain other pharmaceutical additives such as flavoring agents, preservatives, stabilizers, emulsifying agents and buffers. These agents are added in accordance with accepted practices of pharmaceutical compounding.

The compositions provided herein deliver a therapeutically effective dose of ibudilast. In this context, a therapeutically effective dose of ibudilast will depend on the severity of the condition being treated, the medical history of the patient being treated, the age and weight of the person undergoing treatment and will typically range from a dose of about 3 mg/day to about 300 mg/day.

The dosing regimen can be altered by the attending physician depending on the patient's needs. Treatment methods encompass the administration of ibudilast as a single dose, as two daily doses, as three daily doses, as four daily doses, as five daily doses for a time course of one day to several days, weeks, months, and even years. Exemplary dosing schedules include, without limitation, administration five times a day, four times a day, three times a day, twice daily, once daily, every other day, three times weekly, twice weekly, once weekly, twice monthly, once monthly, and so forth.

In some embodiments, ibudilast is administered daily as a single therapeutically effective dose. Exemplary daily doses include without limitation, a dose of about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, or about 300 mg.

For certain treatment regimens, ibudilast is administered as two daily doses with each dose containing about 3 - 150 mg of ibudilast. For instance, each dose can contain about 5 mg of ibudilast, about 10 mg of ibudilast, about 20 mg of ibudilast, about 30 mg of ibudilast, about 40 mg of ibudilast, about 50 mg of ibudilast, about 60 mg of ibudilast, about 70 mg of ibudilast, about 80 mg of ibudilast, about 90 mg of ibudilast, about 100 mg of ibudilast, about 110 mg of ibudilast, about 120 mg of ibudilast, about 130 mg of ibudilast, about 140 mg of ibudilast, or about 150 mg of ibudilast.

### EXAMPLES

### Example 1. Evaluation of ibudilast as a therapeutic agent for decreasing voluntary alcohol consumption in mice

### Example 1-A

The ability of ibudilast (MN-166) to decrease voluntary ethanol consumption, under blind testing conditions, in selectively-bred alcohol-preferring (P) and high-alcohol-drinking (HAD1) rats and in a mouse model of ethanol dependence was examined. While each model is characterized by elevated alcohol intake, drugs such as quetiapine and levatiracetam do not selectively reduce ethanol drinking in these models.

To perform the study, adult male P and HAD1 rats were randomly assigned to receive one of the following four doses of ibudilast - 0 mg/Kg, 3 mg/Kg, 6 mg/Kg, or 9 mg/Kg with eight rats assigned to each dose. Each dosing group was balanced to average a 2 h/day ethanol (15% v/v) intake. Water was concurrently available to the rats and Mazola corn oil was used as the vehicle.

The study was divided into four test phases. In the maintenance test phase rats in the treatment group were injected ibudilast subcutaneously (2 mL/kg s.c.), using standard solutions that deliver 3 mg/Kg, 6 mg/Kg or 9 mg/Kg of ibudilast, 60 min before each ethanol test session and the same dose of ibudilast was administered subcutaneously to the rats 8 hours later. Rats in the control group, however, are administered 2 mL/Kg Mazola corn oil subcutaneously. This protocol was carried out for 4 consecutive days. Following the maintenance test phase, is a two week no drug recovery phase during which rats are permitted access to ethanol and the amount of ethanol consumed by the rats was measured.

The recovery phase is followed by a two week forced ethanol abstinence phase. Following forced abstinence, the effects of ibudilast on ethanol drinking were examined by re-introducing ethanol to rats for a period of 5 consecutive days (i.e., the Relapse Test phase). Each animal received the same dose of ibudilast during maintenance and relapse test phases.

**FIG. 1** illustrates the results of this study. Thus, it is observed that ibudilast reduced ethanol intake by approximately 50% in both P and HAD1 rats **(****FIG. 1**, left panel), during the maintenance test phase. Separate 2-way mixed ANOVA analysis indicates a dose dependent reduction of ethanol intake for P [F(3,28)= 12.425, p< 0.001] and HAD1 [F(3,28)= 14.943, p< 0.001] rats. Each of the four doses of ibudilast reduced ethanol intake over the 4-day maintenance test-phase relative to vehicle-injected controls (p's≤ 0.001).

Ibudilast also reduced ethanol intake in P and HAD1 rats by about 50% during the 5-day Relapse Test phase (**FIG. 1**, right panel). Separate 2-way mixed ANOVA analysis indicates a dose dependent reduction in ethanol intake for P rats [F(3,28)= 8.483, p< 0.001], with the 6 mg/Kg and the 9 mg/Kg doses significantly reducing ethanol drinking relative to controls (p< 0.05), and HAD1 rats [F(3,28)= 25.801, p< 0.001]. Indeed, each test dose of ibudilast was found to reduce ethanol intake compared to controls (p < 0.05).

Significant Dose x Day interactions in P [F(12,112)= 3.257, p< 0.001] and HAD1 [F(12,112)= 2.094, p= 0.023] lines indicated that reduced ethanol intake by ibudilast was most robust on the first day when the drug was administered (data not shown).

### Example 1-B

In a separate study, adult male C57BL/6J mice were used to evaluate ibudilast as a therapeutic for the treatment of alcohol dependence. Two groups of mice were used for the study. Mice in the first group were trained to drink ethanol using a 2 h/day free-choice (15% v/v ethanol) drinking procedure (ethanol dependent (EtOH) mice), while mice in the second group were provided water and served a control (ethanol nondependent (CTL)) mice. Both the ethanol dependent and ethanol non-dependent groups had equal number of mice (N= 37-38/group).

Mice in the alcohol dependent group were then exposed to chronic intermittent ethanol (CIE) vapors for 16 hr/day over four days. After a 72 hours forced abstinence period, the ethanol dependent mice were again permitted access to ethanol for 2 hours each day over a 5 consecutive test days. This pattern of chronic intermittent ethanol vapor exposure for four consecutive days followed by forced abstinence and a five day ethanol access test period was repeated for 9 cycles. Mice in the control (CTL) group were treated in a similar manner except that air was used in the inhalation chambers in place of ethanol vapors.

Both control and ethanol dependent mice received subcutaneous (s.c.) injections of the vehicle at 9 hours and 1 hour prior to the start of daily drinking sessions during Test Cycles 4, 5 and 6 to acclimate the animals to handling and administration of injections.

At the end of test cycle 6, mice in the ethanol dependent group and the control group were separated into four sub-groups with animals receiving one of four doses of ibudilast - 0 mg/Kg, 3 mg/Kg, 6 mg/Kg or 12 mg/Kg during Test Cycles 7 and 8. Each sub-group contained 9-10 mice (N= 9-10/group). The dose of ibudilast was increased during Test Cycle 9 with ethanol dependent and control mice receiving one of the following four doses of ibudilast - 0 mg/Kg, 6 mg/Kg, 12 mg/Kg, or 18 mg/Kg. Thus, mice previously receiving a dose of 3 mg were administered ibudilast at a dose of 18 mg/Kg during Test Cycle 9.

It was observed that ethanol intake by mice increased with successive exposures to chronic intermittent ethanol vapors. For control mice, however, ethanol intake remained relatively the same throughout the nine Test Cycles of the study. Thus, alcohol dependent mice consumed significantly greater quantities of ethanol than control mice starting at Test Cycle 6 (main effect of Group [F(1,67)= 35.84, p< 0.001), and this effect persisted during Test Cycle 7 [F(1,67)= 21.80, p< 0.001], Test Cycle 8 [F(1,67)= 12.52, p< 0.001], and Test Cycle 9 [F(1,66)= 32.55, p< 0.001].

Interestingly, the therapeutic efficacy of ibudilast to lower ethanol consumption appeared to increase with repeated cycles of exposure to chronic intermittent ethanol vapors and drug treatment. As illustrated in Figure 2, ibudilast at a dose of 12 mg/Kg reduced ethanol in alcohol dependent mice. The same dose of ibudilast reduced ethanol consumption to a greater extent during Test Cycle 8, [F(3,67)= 3.36, p< 0.05] in both alcohol dependent and control mice relative to mice receiving vehicle (0 mg of ibudilast) in the alcohol dependent group and the control group respectively (p< 0.05).

The data in **FIG. 2** further illustrates that during Test Cycle 9 ibudilast at a dose of 12 mg/Kg and 18 mg/Kg was more effective at reducing ethanol intake in alcohol dependent mice than control mice (Group x Dose interaction: [F(3,66)= 4.50, p< 0.01]). Pair-wise comparisons indicated that alcohol dependent mice treated with vehicle or 6 mg ibudilast consumed significantly more ethanol than their respective counterparts in the control group (p< 0.05). In contrast, there was no significant difference in ethanol intake for alcohol dependent mice and control mice receiving ibudilast at a dose of 12 mg/Kg and 18 mg/Kg. These results indicate that ibudilast at a dose of 12 mg/Kg or 18 mg/Kg is effective at reducing ethanol intake in alcohol dependent mice to ethanol intake levels for mice in the control group (**FIG. 2**).

Decreased ethanol intake, moreover, does not result from a general suppression of ingestive behavior. Ibudilast was observed to reduce ethanol drinking in alcohol dependent mice at doses that did not affect ethanol drinking in mice in the control group. Moreover, decreased ethanol intake was associated with significant increases in concurrent water intake. While Ibudilast, especially at the 9 mg/Kg dose, produced transitory reductions in 24-hour food intake, but not water intake in P and HAD rats this effect diminished over the 5-day test phase.

### Example 2. Evaluation of the Safety, Tolerance and Efficacy of Ibudilast for the Treatment of Alcoholism and Depression (according to invention) and/or Dysphoric Mood (comparative)

A Phase 1b laboratory study was conducted to determine the safety, tolerability, and initial efficacy of ibudilast (50 mg BID) in humans as a potential AUD therapeutic agent. Human studies were conducted to test that: (1) ibudilast (50 mg BID) does not adversely alter the cardiovascular response to alcohol administered intravenously; (2) ibudilast (50 mg BID) alters the subjective response to alcohol administered intravenously and alcohol-induced cravings; and (3) ibudilast (50 mg BID) alters stress-induced and cue-induced craving.

To address each of the above mentioned specific aims, a randomized, crossover, double-blind, placebo-controlled study was conducted to determine the safety, tolerability, and efficacy of ibudilast in individuals with current AUD Participants were between the ages of 21 and 65 years; met the DSM-5 diagnostic criteria for AUD; reported drinking at least 48 standard drinks in a 30-day period, during the 90 days before enrollment; and were non-treatment seeking alcohol dependent individuals who consented to an inpatient stay at a medical facility for part of the study.

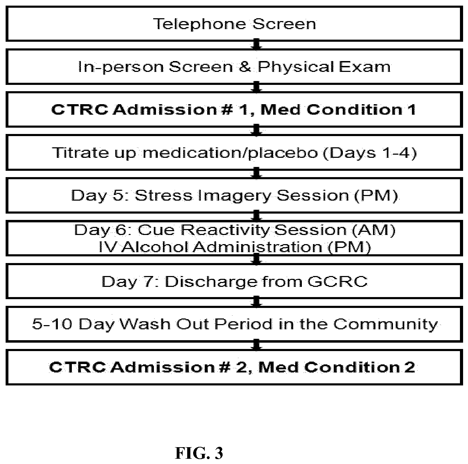

Initially, each subject answered questions about quantity and frequency of drinking. Preliminary screening was conducted by telephone using the Time Line Follow Back (TLFB) protocol. During the screening calls, subjects were asked to provide smoking and drug use data. The present inventors developed a medical history interview questionnaire to screen for medical conditions that contraindicate study participation. Following review of each participant's history along with laboratory tests by the study physician, participants underwent a physical examination where vital signs, weight, and other parameters were measured. Blood and urine samples were collected to test: (a) liver function, (b) glucose levels, (c) presence of drugs, and (d) blood chemistry. Female participants consented to a urine pregnancy screen which was performed prior to each intravenous alcohol administration. The flow chart in **FIG. 3** illustrates the study protocol.

Twenty four non-treatment-seeking individuals with AUD were treated with ibudilast (50 mg BID) and placebo (PBO). Participants completed two alcohol IV challenges, one after taking IBUD for 6 days and one after taking matched PBO for 6 days with a 5-10 day washout period between the study drug conditions. Characteristics of the participants is shown in Table 1 below. Ibudilast was obtained in the form of capsules containing a delayed-release 10 mg formulation. Matching capsules of the placebo also were obtained. Each participant visited the Clinical and Translational Research Center (CTRC) during on Days 1-5 of each medication condition during which they came in for daily morning visits with the study nurse to take the morning study medication under direct observation and took home the evening study medication. The participants stayed overnight on Day 6 and were discharged on Day 7. During the daily visits to the CTRC, the study nurse collected vital signs, asked about side effects in an open-ended fashion and used the Systematic Assessment for Treatment Emergent Events (SAFTEE).

| **Table 1: Sample Characteristics** | | |
|---|---|---|
| | | **Mean (SD) or %** |
| Age | | 31.55 (9.25) |
| Sex - % Male | | 72.7% |
| Ethnicity | | |
| | % Caucasian | 22.70% |
| | % African American | 36.40% |
| Drinking Days per Month | | 20.91 (6.10) |
| Drinks per Drinking Day | | 6.64 (4.21) |
| AUD Symptom Count | | 4.86 (2.61) |
| | % Mild AUD | 28.57% |
| | % Moderate AUD | 38.10% |
| | % Severe AUD | 28.57% |

The study comprised a treatment group and a placebo group and each participant completed a daily assessment questionnaire to address changes in mood, withdrawal effects, alcohol cravings, and address issues pertaining to other side effects. Thus, non-treatment seeking alcohol dependent individuals were treated with ibudilast 50 mg (5 x 10 mg capsules) twice daily (BID) or placebo (PBO). To minimize nausea, however, each participant in the test group received 20 mg of ibudilast twice a day (bid) on days 1 and 2 of the study. The dose was increased to two separate doses of 50 mg each on days 3-6. Upon reaching a stable target dose of ibudilast (or placebo), participants completed a stress-exposure paradigm on day 5 (PM), an alcohol cue-exposure on day 6 (AM), and an intravenous alcohol challenge on day 6 (PM). Following the intravenous alcohol challenge, participants were monitored for 24 hours and then discharged. Following a 5-10 day washout period study participants were readmitted to the CTRC where they received the opposite treatment (IBUD or PBO) for the second part of the study. See Figure 3.

### a. Intravenous Alcohol Administration (IV-A)

Infusion was performed by the study nurse under the supervision of the study physician using a 6% alcohol IV infusion. An alcohol infusion nomogram provides a formula for obtaining the rate of infusion based on the participant's gender and weight. Thus, male participants' were infused at a rate determined by the following formula: 0.166-ml/minute X weight in kilogram while the rate of infusion for female participants was calculated as 0.126-ml/minute X weight in kilograms.

Breath alcohol content (BrAC) was monitored every 3 to 5 minutes during infusion until target BrAC's of .02 g.dl, .04 g.dl, .06 g.dl, and .08 g.dl were obtained, following which infusion rates were reduced to half the original rate to maintain stable BrAC levels during the testing phase. The following parameters were assessed prior to intravenous alcohol challenge and again at each target BrAC: (1) Biphasic Alcohol Effects Scale (BAES) to obtain a reliable and valid measure of alcohol's stimulant and sedative effects; (2) Alcohol Urge Questionnaire (AUQ) - an 8-item scale where subjects rate their craving for alcohol at the present moment, which is appropriate for examining the level of the urge to drink alcohol; (3) Alcohol Ratings Scale, which captures "liking" and "wanting" of alcohol"; (4) The Profile of Mood States (POMS; McNair 1971) which was used to record positive and negative mood states; and (5) Heart Rate and Blood Pressure levels measure at baseline and at each target level of BrAC.

### b. Cue Reactivity Assessment (CR)

The present study also assessed the reactivity of participants to alcohol cues at each dose of medication such that participants served as their own controls. Repeated cue assessments may be useful because studies have indicated that not all alcohol dependent patients are cue reactive. The CR assessments followed well-established procedures and participants received standardized instructions about the assessment as they became acclimated to the psychophysiological monitors.

CR assessment sessions began with a 3-minute relaxation period, in which participants were asked to sit quietly and do nothing. Participants then held and smelled a glass of water for 3 minutes as a standard procedure to control for the effects of simple exposure to any potable liquid, followed by a second 3-minute relaxation period. Next, each participant held and smelled a glass of their preferred alcoholic beverage for three minutes. During each trial, the participant was asked to sniff the beverage for 5 consecutive seconds upon hearing a tone sound. Thirteen tone sounds were administered during each 3-minute block of time. The intervals between tone sounds were varied to ensure that each participant receives the same olfactory exposure. The order in which participants sniffed water or an alcoholic beverage of choice was not counterbalanced because of carryover effects that are known to occur and that would interfere with determination of CR. The water trial provided a baseline that controls for all aspects of stimuli and movement except the nature of the beverage. Participants were allowed a smoke break immediately prior to and immediately after the cue reactivity assessment.

Following each 3 minute exposure to alcohol, each participant rated his/her urge to drink alcohol using the 11-point Likert scale (*e.g.*, "none at all" to "extremely strong urge"). Heart rate (beats per minute; BPM) and blood pressure (systolic, diastolic, and mean arterial pressure [MAP]) were monitored continuously using a Dynamap Adult Vital Signs Monitor and values were averaged over each 3-minute trial. Therapeutic efficacy of ibudilast was gauged based on the compound's ability to attenuate or abolish cue-induced cravings for alcohol consumption.

### c. Stress Reactivity Assessment (SR)

Personal information collected at the time of admission (day 1) to the CRTC was used to generate personalized scripts of neutral and stressful conditions. Participants were asked to identify a recent stressful experience and to rate them on a 0 to 10 Likert scale, where a rating of 10 is considered to be the most stressful experience. Only stressful events rated ≥ 8 were used in script development. Data on physical symptoms associated with the stressful and neutral events were also collected for script development.

During the study, each exposure consisted of 5-minute tape-recorded scripts recounting the recent stressful (or neutral) events in the participants' lives, including cognitions and physical feelings. Stress and neutral conditions were randomized, counterbalanced and a two-hour interval between conditions avoided carryover effects.

The following measures of mood, urge to drink, and physiological reactivity were administered at baseline and post-imagery for both the stress and neutral conditions: (1) The Profile of Mood States, Short Version (POMS) collected data on (a) positive and negative mood; (2) The Alcohol Urge Questionnaire (AUQ) measured subjective levels of alcohol cravings at the present moment (*i.e.*, following stress imagery); (3) Cortisol levels were assessed by collecting saliva samples and heart rate and blood pressure were measured using a Dynamap Adult Vital Signs Monitor. Therapeutic efficacy of ibudilast was gauged based on the compound's ability to attenuate or abolish stress induced cravings for alcohol consumption.

### Results

### Safety

Ibudilast was well tolerated and safe during the study. No subjects dropped out of the study for adverse event-related reasons and there were no dose reductions of ibudilast during the course of the study. Out of the 24 potential adverse drug effects, only headache was reported in significantly greater frequency during the ibudilast vs. placebo regimens (p < 0.05); specifically 4 vs. 0 instances). In the context of alcohol consumption, ibudilast did not affect heart rate or blood pressure either as a main effect or as a moderator of alcohol's effects across the BrACs (p's ≥ 0.35).

### Daily Craving

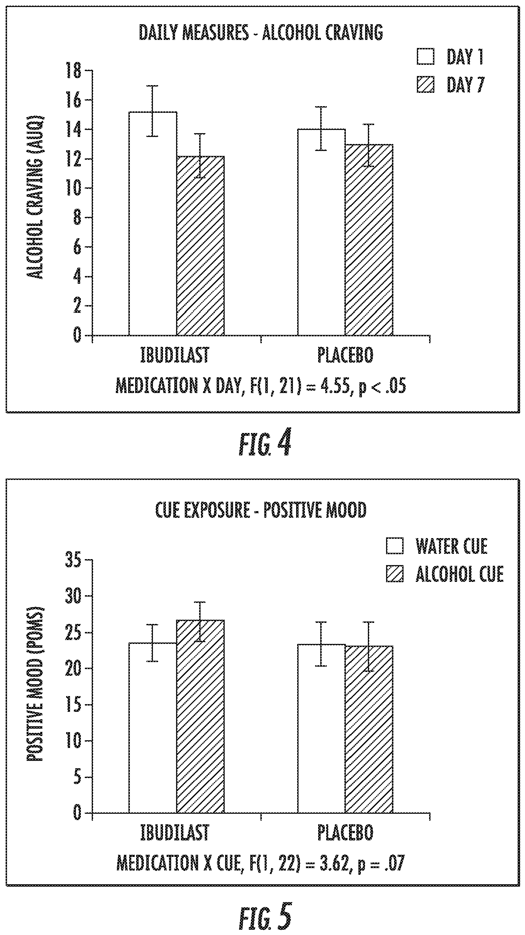

Ibudilast, but not placebo, significantly decreased basal, daily alcohol craving (AUQ) over the course of the study (p < 0.05, see **FIG. 4**).

### Alcohol Cue and Stress Reactivity

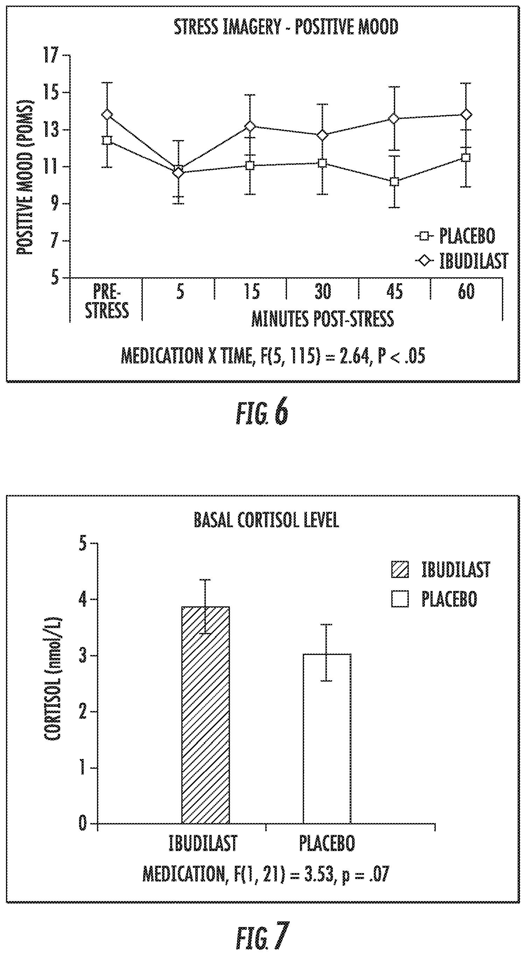

Ibudilast did not affect cue- and stress- induced AUQ craving. However, ibudilast, but not placebo, increased positive mood during both the cue reactivity (p = 0.07, see **FIG. 5**) and stress procedures (p < 0.05; **FIG. 6**). Moreover, ibudilast did not affect cortisol reactivity to the stressor, but ibudilast did produce a modest increase in cortisol level vs. placebo (p = 0.07, see **FIG. 7**).

### Alcohol Infusion

Ibudilast did not affect subjective response to alcohol during the infusion.

These results indicate that ibudilast may be useful for treating AUD by enhancing positive mood, which could ameliorate the mood dysfunction often observed during protracted withdrawal. These findings are also consistent with a hypothesized role for neuro-inflammation in mood dysfunction.

### Post-Hoc Analysis

The results indicated that ibudilast produced a sustained elevation in mood during exposure to alcohol-related cues and stressful imagery. This was not surprising because AUD and depression often co-exist. Moreover, psychoneuroimmunology reports have shown a link between pro-inflammatory cytokines and negative affect and depression. It was possible that MN-166, through its mechanistic actions on pro-inflammatory cytokines, may have enhanced mood and consequently, altered subjective response to alcohol.

To probe the possible relationship between MN-166, mood, and subjective responses to alcohol, further analysis was performed. The following variables were explored in all participants:
1. Baseline scores for depressive symptomology (as measured by the Beck Depression Inventory (BDI)) were collected.
2. Responses to Alcohol Challenge (e.g., alcohol-induced stimulation, positive affect, negative affect, "liking," and "wanting") were compared between subjects with higher BDI scores (more depressive symptomology) and dysphoria and subjects with lower BDI scores (less depressive symptomology) and dysphoria.

The study results showed that subjects with higher BDI scores who were exposed to MN-166 showed less positive and more negative responses to the alcohol challenge, including alcohol-induced stimulation, positive affect, and negative affect, than the subjects with higher BDI scores who were exposed to placebo and the subjects with lower BDI scores. These results, summarized in Table 2 below, indicate that MN-166, through its effects on pro-inflammatory cytokines, benefits individuals with co-morbid diseases such as AUD and depression.

**Table 2**

| Beck Depression Symptoms at Baseline | Study Drug Treatment | Response to Alcohol Challenge¹ | Conclusion |
|---|---|---|---|
| ↑ BDI scores (ie, scored higher on depressive symptoms) | PBO | more positive, less negative | Pro-inflammation may play a role in negative affect and depressive symptoms. An antiinflammatory agent (eg, ibudilast) may modulate the negative affect and depressive symptoms in AUD subjects who scored high on BDI. |
| | IBUDILAST | less positive, more negative | |
| | | | |

| | | | |
|---|---|---|---|
| ¹ Responses measured included: alcohol-induced stimulation, positive affect, negative affect, "liking", and "wanting" | | | |

The medication order (ibudilast first vs. placebo first) effects were evaluated and it was found that medication order did not alter the results of pre-post medication effects on alcohol-related measures as well as stress- and cue-exposure tests. Additionally, mood and craving scores were compared on Day 1 of the first and second medication. It was determined that regardless of medication order, these were not found to be significantly different.

While certain embodiments have been illustrated and described, it should be understood that changes and modifications can be made therein in accordance with ordinary skill in the art without departing from the technology in its broader aspects as defined in the following claims.

The embodiments, illustratively described herein may suitably be practiced in the absence of any element or elements, limitation or limitations, not specifically disclosed herein. Thus, for example, the terms "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the claimed technology.

The present disclosure is not to be limited in terms of the particular embodiments described in this application. Many modifications and variations can be made without departing from its spirit and scope, as will be apparent to those skilled in the art. Functionally equivalent methods and compositions within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds compositions or biological systems, which can of course vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member.

Other embodiments are set forth in the following claims.

## Claims

1. Ibudilast or a pharmaceutically acceptable salt thereof for use in a method of treating a patient diagnosed with alcohol use disorder (AUD), said method comprising administering to a patient diagnosed with AUD a therapeutically effective amount of ibudilast or a pharmaceutically acceptable salt thereof, in which the patient also exhibits symptoms of depression.

2. Ibudilast or a pharmaceutically acceptable salt thereof for use according to claim 1, wherein the symptoms indicate that the patient is suffering from a mild depression; from a moderate depression; or from a severe depression.

3. Ibudilast or a pharmaceutically acceptable salt thereof for use according to claim 1, wherein the ibudilast or pharmaceutically acceptable salt thereof is administered orally.

4. Ibudilast or a pharmaceutically acceptable salt thereof for use according to claim 1, wherein the ibudilast or pharmaceutically acceptable salt thereof is administered at least once daily.

5. Ibudilast or a pharmaceutically acceptable salt thereof for use according to claim 1, wherein the therapeutically effective amount of ibudilast or a pharmaceutically acceptable salt thereof is from about 3 mg to about 200 mg per day.

6. Ibudilast or a pharmaceutically acceptable salt thereof for use according to claim 1, wherein the therapeutically effective amount is administered as a single dose or is divided into two, three, or four doses.

7. Ibudilast or a pharmaceutically acceptable salt thereof for use according to claim 1, wherein the therapeutically effective amount is about 100 mg/day or less and is divided into two or more doses.

8. Ibudilast or a pharmaceutically acceptable salt thereof for use according to claim 1, wherein the therapeutically effective amount is 100 mg/day and is divided into two or more doses.

9. Ibudilast or a pharmaceutically acceptable salt thereof for use according to claim 1, wherein the therapeutically effective amount is about 200 mg/day or less and is divided into two or more doses.

10. Ibudilast or a pharmaceutically acceptable salt thereof for use according to claim 1, wherein the therapeutically effective amount is 200 mg/day and is divided into two or more doses.

11. Ibudilast or a pharmaceutically acceptable salt thereof for use according to claim 1, wherein the ibudilast or a pharmaceutically acceptable salt thereof is administered as part of a combination therapy that includes one or more other therapeutic agents.

12. Ibudilast or a pharmaceutically acceptable salt thereof for use according to claim 11, wherein the one or more other therapeutic agent is a psychiatric therapeutic agent.

13. Ibudilast or a pharmaceutically acceptable salt thereof for use according to claim 12, wherein the psychiatric therapeutic agent is lithium, sertraline, citalopram, carbamazepine, amisulpride, clomipramine, gabapentin, amitriptyline, doxepin, lamotrigine, amoxapine, escitalopram, levetiracetam, agomelatin, fluoxetine, bupropion, fluvoxamine, oxcarbazepine, imipramine, topiramate, clomipramine, mirtazapine, sodium valproate, desipramine, paroxetine, divalproex sodium, desvenlafaxine, sodium valproate, duloxetine, escitalopram, moclobemide, nortriptyline, phenelzine, reboxetine, tianeptine, tranylcypromine, trazodone, venlafaxine, vilazodone, vortioxetine, or any combination of one or more of the foregoing.

14. Ibudilast or a pharmaceutically acceptable salt thereof for use according to claim 11, wherein the one or more other therapeutic agent is approved and known to have a utility in AUD.

15. Ibudilast or a pharmaceutically acceptable salt thereof for use according to claim 14, wherein the therapeutic agent approved and known to have a utility in AUD is acamprosate, baclofen, naltrexone, quetiapine, disulfiram, ondansetron, varenicline, topiramate, prazocin, sertraline, and levatiracetam.

16. Ibudilast or a pharmaceutically acceptable salt thereof for use according to claim 15, wherein therapeutic agent approved and known to have a utility in AUD is naltrexone.

## Patentansprüche

1. Ibudilast oder ein pharmazeutisch annehmbares Salz davon zur Anwendung in einem Verfahren der Behandlung eines Patienten, der mit Alkoholkonsumstörung (AUD) diagnostiziert ist, wobei besagtes Verfahren das Verabreichen einer therapeutisch wirksamen Menge an Ibudilast oder einem pharmazeutisch annehmbaren Salz davon an einen Patienten, der mit AUD diagnostiziert ist, umfasst, wobei der Patient ebenfalls Symptome von Depression vorweist.

2. Ibudilast oder ein pharmazeutisch annehmbares Salz davon zur Anwendung gemäß Anspruch 1, wobei die Symptome indizieren, dass der Patient unter einer leichten Depression; unter einer mittelschweren Depression; oder unter einer schweren Depression leidet.

3. Ibudilast oder ein pharmazeutisch annehmbares Salz davon zur Anwendung gemäß Anspruch 1, wobei das Ibudilast oder pharmazeutisch annehmbare Salz davon oral verabreicht wird.

4. Ibudilast oder ein pharmazeutisch annehmbares Salz davon zur Anwendung gemäß Anspruch 1, wobei das Ibudilast oder pharmazeutisch annehmbare Salz davon zumindest einmal täglich verabreicht wird.

5. Ibudilast oder ein pharmazeutisch annehmbares Salz davon zur Anwendung gemäß Anspruch 1, wobei die therapeutisch wirksame Menge an Ibudilast oder einem pharmazeutisch annehmbaren Salz davon von etwa 3 mg bis etwa 200 mg pro Tag ist.

6. Ibudilast oder ein pharmazeutisch annehmbares Salz davon zur Anwendung gemäß Anspruch 1, wobei die therapeutisch wirksame Menge als eine Einzeldosis verabreicht wird oder in zwei, drei oder vier Dosen aufgeteilt wird.

7. Ibudilast oder ein pharmazeutisch annehmbares Salz davon zur Anwendung gemäß Anspruch 1, wobei die therapeutisch wirksame Menge etwa 100 mg/Tag oder weniger ist und in zwei oder mehr Dosen aufgeteilt wird.

8. Ibudilast oder ein pharmazeutisch annehmbares Salz davon zur Anwendung gemäß Anspruch 1, wobei die therapeutisch wirksame Menge 100 mg/Tag ist und in zwei oder mehr Dosen aufgeteilt wird.

9. Ibudilast oder ein pharmazeutisch annehmbares Salz davon zur Anwendung gemäß Anspruch 1, wobei die therapeutisch wirksame Menge etwa 200 mg/Tag oder weniger ist und in zwei oder mehr Dosen aufgeteilt wird.

10. Ibudilast oder ein pharmazeutisch annehmbares Salz davon zur Anwendung gemäß Anspruch 1, wobei die therapeutisch wirksame Menge 200 mg/Tag ist und in zwei oder mehr Dosen aufgeteilt wird.

11. Ibudilast oder ein pharmazeutisch annehmbares Salz davon zur Anwendung gemäß Anspruch 1, wobei das Ibudilast oder ein pharmazeutisch annehmbares Salz davon als Teil einer Kombinationstherapie verabreicht wird, die eine oder mehr andere Therapeutika beinhaltet.

12. Ibudilast oder ein pharmazeutisch annehmbares Salz davon zur Anwendung gemäß Anspruch 11, wobei das eine oder mehr andere Therapeutikum ein psychiatrisches Therapeutikum ist.

13. Ibudilast oder ein pharmazeutisch annehmbares Salz davon zur Anwendung gemäß Anspruch 12, wobei das psychiatrische Therapeutikum Lithium, Sertralin, Citalopram, Carbamazepin, Amisulprid, Clomipramin, Gabapentin, Amitriptylin, Doxepin, Lamotrigin, Amoxapin, Escitalopram, Levetiracetam, Agomelatin, Fluoxetin, Bupropion, Fluvoxamin, Oxcarbazepin, Imipramin, Topiramat, Clomipramin, Mirtazapin, Natriumvalproat, Desipramin, Paroxetin, Divalproex-Natrium, Desvenlafaxin, Natriumvalproate, Duloxetin, Escitalopram, Moclobemid, Nortriptylin, Phenelzin, Reboxetin, Tianeptin, Tranylcypromin, Trazodon, Venlafaxin, Vilazodon, Vortioxetin, oder eine Kombination aus einem oder mehr der Vorhergehenden ist.

14. Ibudilast oder ein pharmazeutisch annehmbares Salz davon zur Anwendung gemäß Anspruch 11, wobei das eine oder mehr andere Therapeutikum zugelassen zu und bekannt für das Aufweisen eines Nutzens in AUD ist.

15. Ibudilast oder ein pharmazeutisch annehmbares Salz davon zur Anwendung gemäß Anspruch 14, wobei das Therapeutikum, das zugelassen zu und bekannt für das Aufweisen eines Nutzens in AUD ist, Acamprosat, Baclofen, Naltrexon, Quetiapin, Disulfiram, Ondansetron, Vareniclin, Topiramat, Prazocin, Sertralin, und Levatiracetam ist.

16. Ibudilast oder ein pharmazeutisch annehmbares Salz davon zur Anwendung gemäß Anspruch 15, wobei das Therapeutikum, das zugelassen zu und bekannt für das Aufweisen eines Nutzens in AUD ist, Naltrexon ist.

## Revendications

1. - Ibudilast ou sel pharmaceutiquement acceptable de celui-ci pour une utilisation dans une méthode de traitement d'un patient diagnostiqué avec un trouble lié à la consommation d'alcool (« alcohol use disorder » - AUD), ladite méthode comprenant l'administration à un patient diagnostiqué avec un AUD d'une quantité thérapeutiquement efficace d'ibudilast ou d'un sel pharmaceutiquement acceptable de celui-ci, le patient présentant également des symptômes de dépression.

2. - Ibudilast ou sel pharmaceutiquement acceptable de celui-ci pour une utilisation selon la revendication 1, les symptômes indiquant que le patient souffre d'une dépression légère ; d'une dépression modérée ; ou d'une dépression sévère.

3. - Ibudilast ou sel pharmaceutiquement acceptable de celui-ci pour une utilisation selon la revendication 1, l'ibudilast ou le sel pharmaceutiquement acceptable de celui-ci étant administré par voie orale.

4. - Ibudilast ou sel pharmaceutiquement acceptable de celui-ci pour une utilisation selon la revendication 1, l'ibudilast ou le sel pharmaceutiquement acceptable de celui-ci étant administré au moins une fois par jour.

5. - Ibudilast ou sel pharmaceutiquement acceptable de celui-ci pour une utilisation selon la revendication 1, la quantité thérapeutiquement efficace d'ibudilast ou d'un sel pharmaceutiquement acceptable de celui-ci étant d'environ 3 mg à environ 200 mg par jour.

6. - Ibudilast ou sel pharmaceutiquement acceptable de celui-ci pour une utilisation selon la revendication 1, la quantité thérapeutiquement efficace étant administrée en une seule dose ou étant divisée en deux, trois ou quatre doses.

7. - Ibudilast ou sel pharmaceutiquement acceptable de celui-ci pour une utilisation selon la revendication 1, la quantité thérapeutiquement efficace étant d'environ 100 mg/jour ou moins et étant divisée en deux doses ou plus.

8. - Ibudilast ou sel pharmaceutiquement acceptable de celui-ci pour une utilisation selon la revendication 1, la quantité thérapeutiquement efficace étant de 100 mg/jour et étant divisée en deux doses ou plus.

9. - Ibudilast ou sel pharmaceutiquement acceptable de celui-ci pour une utilisation selon la revendication 1, la quantité thérapeutiquement efficace étant d'environ 200 mg/jour ou moins et étant divisée en deux doses ou plus.

10. - Ibudilast ou sel pharmaceutiquement acceptable de celui-ci pour une utilisation selon la revendication 1, la quantité thérapeutiquement efficace étant de 200 mg/jour et étant divisée en deux doses ou plus.

11. - Ibudilast ou sel pharmaceutiquement acceptable de celui-ci pour une utilisation selon la revendication 1, l'ibudilast ou un sel pharmaceutiquement acceptable de celui-ci étant administré en tant que partie d'une thérapie de combinaison qui comprend un ou plusieurs autres agents thérapeutiques.

12. - Ibudilast ou sel pharmaceutiquement acceptable de celui-ci pour une utilisation selon la revendication 1, le ou les autres agents thérapeutiques étant un agent thérapeutique psychiatrique.

13. - Ibudilast ou sel pharmaceutiquement acceptable de celui-ci pour une utilisation selon la revendication 12, dans lequel l'agent thérapeutique psychiatrique est le lithium, la sertraline, le citalopram, la carbamazépine, l'amisulpride, la clomipramine, la gabapentine, l'amitriptyline, la doxépine, la lamotrigine, l'amoxapine, l'escitalopram, le lévétiracétam, l'agomélatine, la fluoxétine, le bupropion, la fluvoxamine, l'oxcarbazépine, l'imipramine, le topiramate, la clomipramine, la mirtazapine, le valproate de sodium, la désipramine, la paroxétine, le divalproex sodium, la desvenlafaxine, le valproate de sodium, la duloxétine, l'escitalopram, le moclobémide, la nortriptyline, la phénelzine, la réboxétine, la tianeptine, la tranylcypromine, la trazodone, la venlafaxine, la vilazodone, la vortioxétine, ou toute combinaison d'un ou plusieurs des agents thérapeutiques précédents.

14. - Ibudilast ou sel pharmaceutiquement acceptable de celui-ci pour une utilisation selon la revendication 11, le ou les autres agents thérapeutiques étant approuvés et connus pour avoir une utilité dans l'AUD.

15. - Ibudilast ou sel pharmaceutiquement acceptable de celui-ci pour une utilisation selon la revendication 14, l'agent thérapeutique approuvé et connu pour avoir une utilité dans l'AUD étant l'acamprosate, le baclofène, la naltrexone, la quétiapine, le disulfiram, l'ondansétron, la varénicline, le topiramate, la prazocine, la sertraline et le lévétiracétam.

16. - Ibudilast ou sel pharmaceutiquement acceptable de celui-ci pour une utilisation selon la revendication 15, l'agent thérapeutique approuvé et connu pour avoir une utilité dans l'AUD étant la naltrexone.
